(19) Europäisches Patentamt
European Patent Office
Office européen des brevets

(11) Publication number: **0 008 746**
**B1**

(12) **EUROPEAN PATENT SPECIFICATION**

(45) Date of publication of patent specification: **11.08.82**

(21) Application number: **79103092.7**

(22) Date of filing: **22.08.79**

(51) Int. Cl.³: **C 07 D 401/12,**
A 61 K 31/47 //C07D211/60,
(C07D401/12, 215/36,
211/60)

(54) **Alpha-(N-arylsulfonyl-L-argininamides, processes for their preparation and pharmaceutical compositions containing these substances.**

(30) Priority: **31.08.78 US 938711**
**22.05.79 US 41419**

(43) Date of publication of application:
**19.03.80 Bulletin 80/6**

(45) Publication of the grant of the patent:
**11.08.82 Bulletin 82/32**

(84) Designated Contracting States:
**AT BE CH DE FR GB IT LU NL SE**

(56) References cited:
**DE - A - 2 801 478**

The file contains technical information submitted after the application was filed and not included in this specification.

(73) Proprietor: **MITSUBISHI CHEMICAL INDUSTRIES LIMITED**
**5-2, Marunouchi 2-chome Chiyoda-ku**
**Tokyo 100 (JP)**

(73) Proprietor: **Okamoto, Shosuke**
**15-18, Asahigaoka 3-chome Tarumi-ku**
**Kobe-shi Hyogo (JP)**

**EP 0 008 746 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Kikumoto, Ryoji**
**1521-29, Nogaya-cho Machida-shi**
**Tokyo (JP)**
Inventor: **Tamao, Yoshikuni**
**4402, Naruse Machida-shi**
**Tokyo (JP)**
Inventor: **Ohkubo, Kazuo**
**922, Inokata Komae-shi**
**Tokyo (JP)**
Inventor: **Tezuka, Tohru**
**122 Lawn Terrace, Apt. 2H**
**Mamaroneck New York 10543 (US)**
Inventor: **Tonomura, Shinji**
**17-6, Tsurukawa 5-chome Machida-shi**
**Tokyo (JP)**
Inventor: **Hijikata, Akiko**
**808, 1-13, Isogamidori 5-chome Fukiai-ku**
**Kobe-shi Hyogo (JP)**
Inventor: **Okamoto, Shosuke**
**15-18, Asahigaoka 3-chome Tarumi-ku**
**Kobe-shi Hyogo (JP)**

(74) Representative: **Patentanwälte TER MEER - MÜLLER - STEINMEISTER**
**Triftstrasse 4**
**D-8000 München 22 (DE)**

Alpha-(N-arylsulfonyl-L-argininamides, processes for their preparation and pharmaceutical compositions containing these substances

Background of the invention

*Field of the invention*

This invention relates to the discovery of certain new and useful $N^2$-arylsulfonyl-L-argininamides and the pharmaceutically acceptable salts thereof, which are of especial value in view of their outstanding antithrombotic properties and low toxicities.

*Description of the prior art*

In the past, there have been many attempts to obtain new and improved agents for the treatment of thrombosis. The $N^2$-(p-tolylsulfonyl)-L-arginine esters have been found to be one type of agent which can be used and these have been found to be effective in dissolving blood clots. (U.S. Patent No. 3,622,615, issued November 23, 1971). On family of compounds which have been found to be particularly useful as highly specific inhibitors of thrombin for the control of thrombosis is the $N^2$-dansyl-L-arginine ester or amide (U.S. Patent No. 3,978,045). However, there is a continuing need for a highly specific inhibitor of thrombin for the control of thrombosis, which exhibits lower toxicity.

The compounds of the present case are structural closely related to the $N^2$-arylsulfonyl-L-argininamides disclosed in DE—OS 28 01 428 (A).

*Summary of the invention*

It has now been discovered that $N^2$-arylsulfonyl-L-argininamides exhibit antithrombotic activity and even lower toxicity levels at the same relative potencies, as compared with the $N^2$-dansyl-L-arginine ester or amide.

The present invention provides an $N^2$-arylsulfonyl-L-argininamide of the formula (I):

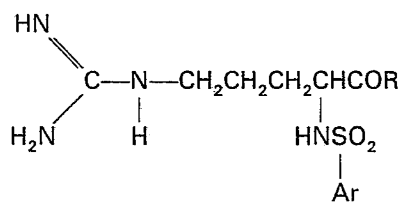

(I)

wherein R is

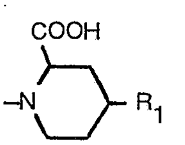

wherein $R_1$ is hydrogen or $C_1$—$C_5$ alkyl; and Ar is 1,2,3,4-tetrahydro-8-quinolyl optionally substituted with at least one $C_1$—$C_5$ alkyl.

Also encompassed within this invention are pharmaceutically acceptable salts thereof.

This invention also relates to a pharmaceutical composition which comprises an antithrombotically effective amount of a compound of the formula (I) and a pharmaceutically acceptable adjuvant.

DETAILED DESCRIPTION OF THE PREFERRED EMBODIMENTS

This invention relates to a group of $N^2$-arylsulfonyl-L-argininamides of the formula (I):

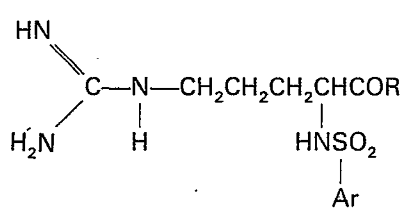

(I)

wherein R is

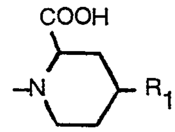

wherein $R_1$ is hydrogen or alkyl of 1—5 (preferably 1—3) carbon atoms; and Ar is 1,2,3,4-tetrahydro-8-quinolyl optionally substituted with at least one (preferably one or two) alkyl of 1—5 (preferably 1—3) carbon atoms.

Typical compounds of this invention include:

1-[N²-(1,2,3,4-tetrahydro-8-quinolinesulfonyl)-L-arginyl]-4-methyl-2-piperidinecarboxylic acid
1-[N²-(1,2,3,4-tetrahydro-8-quinolinesulfonyl)-L-arginyl]-4-ethyl-2-piperidinecarboxylic acid
1-[N²-(3-methyl-1,2,3,4-tetrahydro-8-quinolinesulfonyl)-L-arginyl]-4-methyl-2-piperidinecarboxylic acid
1-[N²-(3-methyl-1,2,3,4-tetrahydro-8-quinolinesulfonyl)-L-arginyl]-4-ethyl-2- piperidinecarboxylic acid
1-[N²-(3-ethyl-1,2,3,4-tetrahydro-8-quinolinesulfonyl)-L-arginyl]-4-methyl-2- piperidinecarboxylic acid
1-[N²-(3-ethyl-1,2,3,4-tetrahydro-8-quinolinesulfonyl)-L-arginyl]-4-ethyl-2-piperidinecarboxylic acid
(2R,4R)-1-[N²-(3-methyl-1,2,3,4-tetrahydro-8-quinolinesulfonyl)-L-arginyl]-4-methyl-2-piperidinecarboxylic acid

Of the compounds of this invention, preferred are those wherein R is (2R,4R)-4-alkyl-2-carboxy-1-piperidino said alkyl being $C_1$—$C_5$ alkyl and Ar is 3-alkyl-1,2,3,4-tetrahydro-8-quinolyl said alkyl being $C_1$—$C_3$ alkyl.

The pharmaceutically acceptable salts of the above compounds are of course also included within the scope of this invention.

For the preparation of the compounds of this invention, various methods can be employed depending upon the particular starting materials and/or intermediates involved. Successful preparation of these compounds is possible by way of a synthetic route which is outlined below.

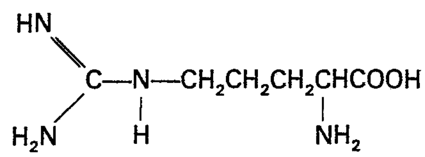

(II) →

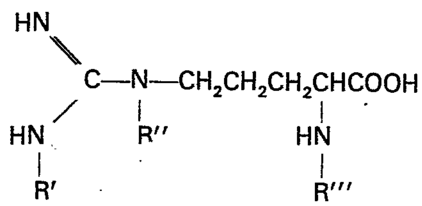

(III)

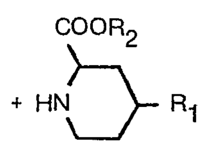

(IV) ⟶

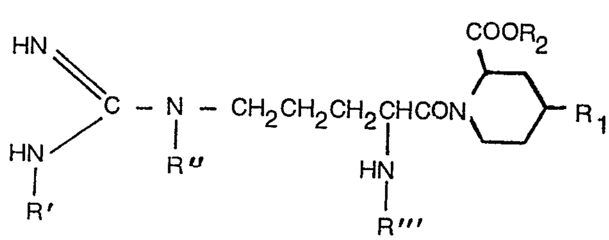

(V) ⟶

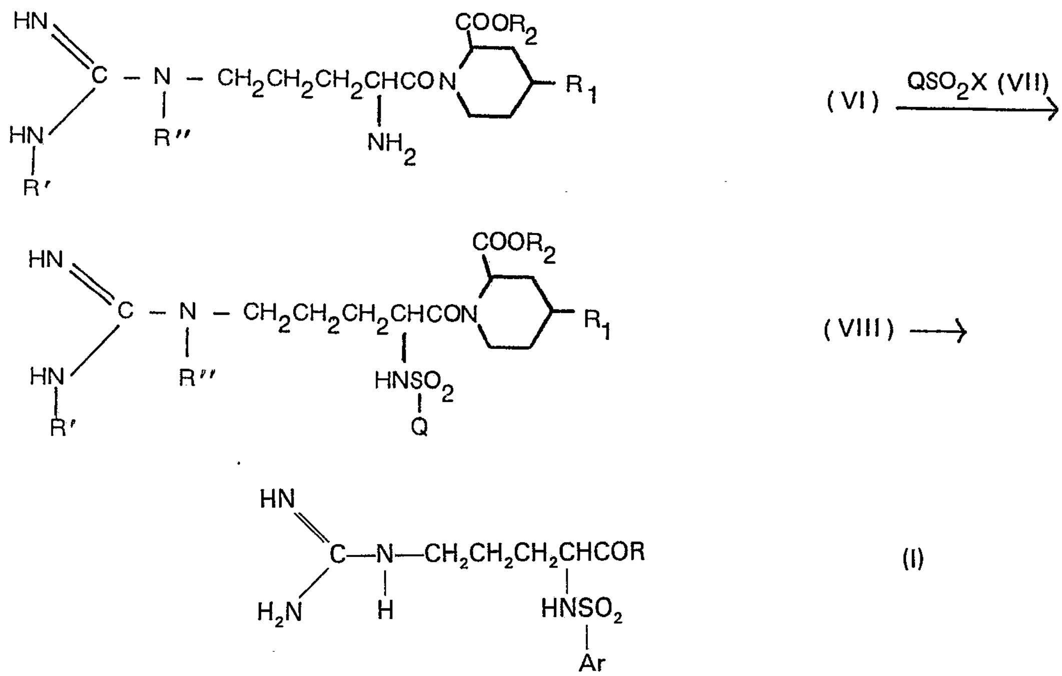


(VI)

(VIII)

(I)

In the above formulas, R, Ar and $R_1$ are as defined herein above; X is halogen; R‴ is a protective group for the $\alpha$-amino group, such as benzyloxycarbonyl or tert-butoxycarbonyl; R′ and R″ are selected from the group consisting of hydrogen and protective groups for the guanidino group, such as nitro, tosyl, trityl, oxycarbonyl and the like; and at least one of R′ and R″ is a protective group for the guanidino group; $R_2$ is hydrogen, lower alkyl of normally 1 to 10 carbon atoms, such as methyl and ethyl, or aralkyl of normally 7 to 15 carbon atoms, such as benzyl and phenethyl; and Q is 8-quinolyl optionally substituted with at least one $C_1$—$C_5$ alkyl, which is corresponding to Ar.

The $N^2$-arylsulfonyl-L-argininamide (I) is prepared by removing the $N^G$-substituent and, when $R_2$ is aralkyl, that aralkyl group from an $N^G$-substituted-$N^2$-quinolinesulfonyl-L-argininamide (VIII) by means of hydrogenolysis and, at the same time, hydrogenating the quinolyl moiety to the corresponding 1,2,3,4-tetrahydroquinolyl moiety and, when $R_2$ is alkyl, hydrolyzing the ester group at the 2 position of the piperidine ring.

The removal of the nitro group and the oxycarbonyl group, e.g., benzyloxycarbonyl, p-nitrobenzyloxycarbonyl, as protective groups for the guanidino group as well as the removal of the aralkyl group as an alcohol moiety of the ester group is readily accomplished by the hydrogenolysis.

The hydrogenolysis and hydrogenation are concurrently effected in a reaction-inert solvent, e.g., alcohols such as methanol, ethanol and the like; or ethers such as tetrahydrofuran, dioxane or the like, in the presence of a hydrogen-activating catalyst, e.g., Raney Nickel, cobalt, or noble metal catalyst such as palladium, platinum, ruthenium, rhodium, and preferably noble metal catalyst, in a hydrogen atmosphere at a temperature of 0°C to 200°C and preferably 30°C to 150°C. The reaction period varies with catalyst, hydrogen pressure and reaction temperature and generally is in the range of 30 minutes to 120 hours.

In general, the hydrogen pressure is in the range of 1 to 200 kg/cm², and preferably in the range of 1 to 100 kg/cm². It is necessary to continue the hydrogenolysis and hydrogenation until a stoichiometric amount of hydrogen is absorbed.

Addition of an organic acid, e.g., acetic acid, propionic acid, or an inorganic acid, e.g., hydrochloric acid accelerates the reaction. The organic acid such as acetic acid can also be used alone as a solvent.

The hydrolysis can be effected at any time, that is, before, during or after the hydrogenolysis and hydrogenation. The hydrolysis of the ester group wherein $R_2$ is alkyl or aralkyl (only in case where the hydrolysis is effected before the hydrogenolysis and hydrogenation) is effected by the conventional process using an acid such as a mineral acid (hydrochloric acid, sulfuric acid or the like) or a base such as inorganic base (sodium hydroxide, potassium hydroxide, barium hydroxide, potassium carbonate).

Alkaline hydrolysis is normally effected in water or an inert organic solvent containing water (e.g., alcohols, tetrahydrofuran, dioxane) at a temperature from 20 to 150°C. The reaction period varies with the reaction condition and normally is in the range of 5 minutes to 20 hours.

Acid hydrolysis is effected in water alone or an inert organic solvent containing water (e.g., alcohols, tetrahydrofuran, dioxane) at a temperature in the range of 20 to 150°C for a period of from 30 minutes to 50 hours.

1-($N^G$-substituted-$N^2$-quinolinesulfonyl-L-arginyl)-2-piperidinecarboxylic acids or 1-$N^G$-substituted-$N^2$-quinolinesulfonyl-L-arginyl)-2-piperidinecarboxylate (VIII) can be prepared by condensing an $N^G$-substituted-$N^2$-substituted L-arginine (III) (generally the $N^G$-substituent is nitro or acyl, and the $N^2$-substituent is a protective group for the amino group, such as benzyloxycarbonyl, tert-butoxycarbonyl, or the like) or its reactive derivative such as an acid halide, acid azide, activated ester or mixed carbonic anhydride and a corresponding amino acid derivative (IV) or its reactive derivative such as a mono- or disilyl derivative and if necessary, in the presence of a condensing agent such as a carbodiimide, selectively removing only the $N^2$-substituent of an $N^G$-substituted-$N^2$-substituted L-argininamide (V) by means of catalytic hydrogenolysis or acidolysis, and then condensing the thus obtained $N^G$-substituted-L-argininamide (VI) with a quinolinesulfonyl halide (VII), preferably a chloride in the presence of a base in a solvent.

Alternatively, the $N^2$-arylsulfonyl-L-argininamide (I) can be prepared by hydrogenating the quinolyl moiety of the $N^2$-quinolinesulfonyl-L-argininamide (X) to the corresponding 1,2,3,4-tetrahydroquinolyl moiety. This process may be illustrated as follows:

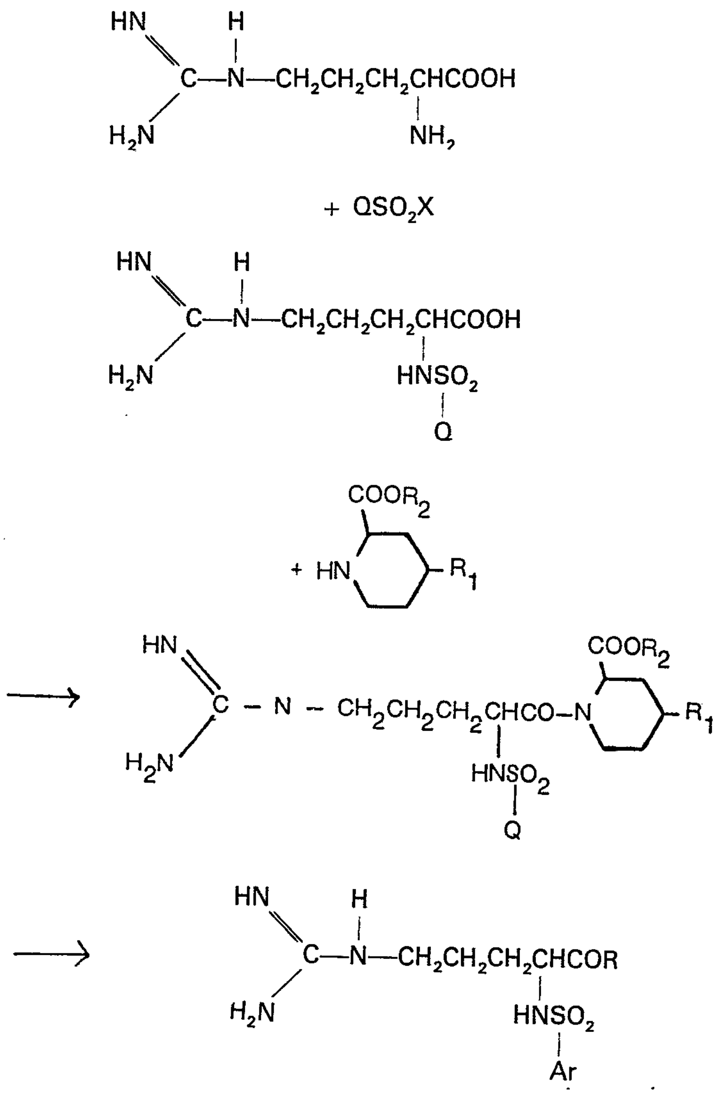

(II)

(VII)

(IX)

(IV)

(X)

(I)

In the above formula, R, $R_1$ $R_2$, Q, Ar and X are as defined herein above.

The $N^2$-arylsulfonyl-L-argininamide (I) is prepared by hydrogenating the quinolyl moiety of the $N^2$-quinolinesulfonyl-L-argininamide (X) and at the same time, when $R_2$ is aralkyl, removing that aralkyl group by means of hydrogenolysis, and when $R_2$ is alkyl, hydrolyzing that alkyl ester.

The conditions under which hydrogenation and hydrogenolysis are carried out are substantially the same as those described in the hydrogenation and hydrogenolysis of 1-($N^G$-substituted-$N^2$-quinolinesulfonyl-L-arginyl)-2-piperidinecarboxylic acids or their esters (VIII).

The $N^2$-quinolinesulfonyl-L-argininamide (X) is prepared by the condensation of an $N^2$-quinolinesulfonyl-L-arginine (IX) or its reactive derivative such as an acid halide, acid azide, activated ester (e.g., p-nitrophenyl ester) or mixed carbonic anhydride, and a corresponding amino acid derivative (IV) or its

reactive derivative such as a mono- or disilyl derivative and if necessary, in the presence of a condensing agent such as a carbodiimide (e.g., 1,3-dicyclohexylcarbodiimide).

It is frequently more convenient and practical to use the reactive derivative of the $N^2$-quinolylsulfonyl-L-arginine (IX) in the form of the solution, in which they are prepared. The conditions under which condensation would be carried out will be each apparent to those skilled in the art.

The $N^2$-quinolinesulfonyl-L-arginine (IX) can be prepared by the condensation of L-arginine (II) with a substantially equimolar amount of a quinolinesulfonyl halide (VII), preferably a chloride in the presence of a base in a solvent.

The $N^2$-arylsulfonyl-L-argininamides (I) thus obtained are isolated and purified by conventional means. For example, they are isolated by filtration of the catalyst followed by evaporation of the solvent, and then purified by trituration or recrystallization from a suitable solvent, such as diethyl ether-tetrahydrofuran, diethyl ether-methanol and water-methanol, or may be chromatographed on silica gel or alumina.

Starting from (2R,4R)-4-alkyl-2-piperidinecarboxylic acids or the esters thereof, (2R,4R)-1-($N^2$-1,2,3,4-tetrahydro-8-quinolinesulfonyl-L-arginyl)-4-alkyl-2-piperidinecarboxylic acids can be prepared by the methods described above.

The $N^2$-arylsulfonyl-L-argininamide (I) of this invention forms acid addition salts with any of a variety of inorganic and organic acids. They also form salts with any of a variety of inorganic and organic bases.

The product of the reactions described above can be isolated in the free form or in the form of salts. In addition, the product can be obtained as pharmaceutically acceptable acid addition salts by reacting one of the free bases with an acid, such as hydrochloric, hydrobromic, hydroiodic, nitric, sulfuric, phosphoric, acetic, citric, maleic, succinic, lactic, tartaric, gluconic, benzoic, methanesulfonic, ethanesulfonic, benzenesulfonic, p-toluenesulfonic acid or the like. In a similar manner, the product can be obtained as pharmaceutically acceptable salts by reacting one of the free carboxylic acids with a base, such as sodium hydroxide, potassium hydroxide, ammonium hydroxide, triethylamine, procaine, dibenzylamine, 1-epheneamine, N,N'-dibenzylethylenediamine, N-ethylpiperidine or the like.

Likewise, treatment of the salts with a base or acid results in a regeneration of the free amide.

As stated above, the $N^2$-arylsulfonyl-L-argininamides, and the salts thereof of this invention are characterized by their highly specific inhibitory activity in mammals against thrombin as well as by their substantial lack of toxicity, and therefore these compounds are useful in the determination of thrombin in blood as diagnostic reagents, and/or for the medical control or prevention of thrombosis.

The compounds of this invention are also useful as an inhibitor of platelet aggregation.

The antithrombotic activity of the $N^2$-arylsulfonyl-L-argininamide of this invention was compared with that of a known antithrombotic agent, $N^2$-(p-tolylsulfonyl)-L-arginine methyl ester, by determining the fibrinogen coagulation time. The measurement of the fibrinogen coagulation time was conducted as follows:

An 0.8 ml aliquot of a fibrinogen solution, which had been prepared by dissolving 150 mg of bovine fibrinogen (Cohn fraction I) supplied by Armour Inc. in 40 ml of a borate saline buffer (pH 7.4), was mixed with 0.1 ml of a borate saline buffer, pH 7.4, (control) or a sample solution in the same buffer, and 0.1 ml of a thrombin solution (5 units/ml) supplied by Mochida Pharmaceutical Co., Ltd. was added to the solutions in an ice bath.

Immediately after mixing, the reaction mixture was transferred from the ice bath to a bath maintained at 25°C.

Coagulation times were taken as the period between the time of transference to the 25°C bath and the time of the first appearance of fibrin threads. In the cases where no drug samples were added, the coagulation time was 50—55 seconds.

The experimental results are summarized in Table 1. The term "concentration required to prolong the coagulation time by a factor of two" is the concentration of an active ingredient required to prolong the normal coagulation time 50—55 seconds to 100—110 seconds.

The concentration required to prolong the coagulation time by a factor of two for the known antithrombotic agent, $N^2$-(p-tolylsulfonyl)-L-arginine methyl ester, was 1,100 $\mu$m. The inhibitors are shown in Table 1 by indicating R and Ar in the formula (I) and the addition moiety.

When a solution containing an $N^2$-arylsulfonyl-L-argininamide of this invention was administered intravenously into animal bodies, the high antithrombotic activity in the circulating blood was maintained for from one to three hours.

The halflife for decay of the anti-thrombotic compounds of this invention in circulating blood was shown to be approximately 60 minutes; the physiological conditions of the host animals (rats, rabbit, dog and chimpanzee) were well maintained. The experimental decrease of fibrinogen in animals caused by infusion of thrombin was satisfactorily controlled by similtaneous infusion of the compounds of this invention.

The acute toxicity values ($LD_{50}$) determined by intravenous administration of substances of formula (I) in mice (male, 20 g) range from about 100 to 500 milligrams per kilogram of body weight.

Representative $LD_{50}$ values for 1-[$N^2$-(1,2,3,4-tetrahydro-8-quinolinesulfonyl)-L-arginyl]-4-methyl-2-piperidinecarboxylic acid, 1-[$N^2$-(3-methyl-1,2,3,4-tetrahydro-8-quinolinesulfonyl)-L-arginyl]-

4 - methyl - 2 - piperidinecarboxylic acid, 1 - [$N^2$ - (3 - ethyl - 1,2,3,4 - tetrahydro - 8 - quinolinesulfonyl)-L - arginyl] - 4 - methyl - 2 - piperidinecarboxylic acid, 1 - [$N^2$ - (1,2,3,4 - tetrahydro - 8 - quinolinesulfonyl) - L - arginyl] - 4 - ethyl - 2 - piperidinecarboxylic acid and (2R,4R) - 1 - $N^2$ - (3 - methyl-1,2,3,4 - tetrahydro - 8 - quinolinesulfonyl) - L - arginyl - 4 - methyl - 2 - piperidinecarboxylic acid are 191, 264, 322, 132 and 210 milligrams per kilogram, respectively.

On the other hand, $LD_{50}$ values for $N^2$-dansyl-N-butyl-L-argininamide and $N^2$-dansyl-N-methyl-N-butyl-L-argininamide are 10 and 5 milligrams per kilogram, respectively.

The therapeutic agents in this invention may be administered to mammals, including humans, alone or in combination with pharmaceutically acceptable carriers, the proportion of which is determined by the solubility and chemical nature of the compound, chosen route of administration and standard pharmaceutical practice.

For example, the compounds may be injected parenterally, that is, intramuscularly, intravenously or subcutaneously. For parenteral administration, the compounds may be used in the form of sterile solutions containing other solutes, for example, sufficient saline or glucose to make the solution isotonic. The compounds may be administered orally in the form of tablets, capsules, or granules containing suitable excipients such as starch, lactose, white sugar and the like.

The compounds may be administered sublingually in the form of troches or lozenges in which each active ingredient is mixed with sugar or corn syrups, flavoring agents and dyes, and then dehydrated sufficiently to make the mixture suitable for pressing into solid form. The compounds may be administered orally in the form of solutions which may contain coloring and flavoring agents. Physicians will determine the dosage of the present therapeutic agents which will be most suitable for humans, and dosages vary with the mode of administration and the particular compound chosen. In addition, the dosage will vary with the particular patient under treatment.

When the composition is administered orally, a larger quantity of the active agent will be required to produce the same effect as caused with a smaller quantity given parenterally.

The therapeutic dosage is generally 10—50 mg/kg of active ingredient parenterally, 10—500 mg/kg orally per day.

Having generally described the invention, a more complete understanding can be obtained by reference to certain specific examples, which are included for purposes of illustration only and are not intended to be limiting unless otherwise specified.

It is to be understood that the present invention includes pharmaceutical compositions containing a compound of the invention as an active ingredient. Such compositions may be in the form described above. In particular, the invention includes such compositions in unit dose form.

## Example 1

(A) Ethyl 1-[$N^G$-nitro-$N^2$-(tert-butoxycarbonyl)-L-arginyl]-4-methyl-2-piperidinecarboxylate

To a stirred solution of 28.3 g of $N^G$-nitro-$N^2$-(tert-butoxycarbonyl)-L-arginine in 450 ml of dry tetrahydrofuran were added in turn 9.0 g of triethylamine and 12.2 g of isobutyl chloroformate while keeping the temperature at −20°C. After 10 minutes, to this was added 15.2 g of ethyl 4-methyl-2-piperidinecarboxylate and the mixture was stirred for 10 minutes at −20°C. At the end of this period, the reaction mixture was warmed to room temperature. The solvent was evaporated and the residue taken up in 400 ml of ethyl acetate, and washed successively with 200 ml of water, 100 ml of 5% sodium bicarbonate solution, 100 ml of 10% citric acid solution and 200 ml of water. The ethyl acetate solution was dried over anhydrous sodium sulfate.

The solution was evaporated to give 31.5 g (75 percent) of ethyl 1-[$N^G$-nitro-$N^2$-(tert-butoxycarbonyl)-L-arginyl]-4-methyl-2-piperidinecarboxylate in the form of a syrup.
I.R. (KBr): 3,300, 1,730, 1,680 $cm^{-1}$

(B) Ethyl 1-[$N^G$-nitro-L-arginyl]-4-methyl-2-piperidinecarboxylate hydrochloride

To a stirred solution of 30 g of ethyl 1-[$N^G$-nitro-$N^2$-(tert-butoxycarbonyl)-L-arginyl]-4-methyl-2-piperidinecarboxylate in 50 ml of ethyl acetate was added 80 ml of 10% dry HCl-ethyl acetate at 0°C. After 3 hours, to this solution was added 200 ml of dry ethyl ether to precipitate a viscous oily product.

This was filtered and washed with dry ethyl ether to give ethyl 1-[$N^G$-nitro-L-arginyl]-4-methyl-2-piperidinecarboxylate hydrochloride as an amorphous solid.

(C) Ethyl 1-[$N^G$-nitro-$N^2$-(3-methyl-8-quinolinesulfonyl)-L-arginyl]-4-methyl-2-piperidinecarboxylate

To a stirred solution of 25 g of ethyl 1-($N^G$-nitro-L-arginyl)-4-methyl-2-piperidinecarboxylate hydrochloride in 200 ml of chloroform were added in turn 18.5 g of triethylamine, and 14.7 g of 3-methyl-8-quinolinesulfonyl chloride at 5°C, and stirring was continued for 3 hours at room temperature. At the end of this period, the solution was washed twice with 50 ml of water.

The chloroform solution was dried over anhydrous sodium sulfate. Upon evaporation of the solvent, the residue was chromatographed on 50 g of silica gel packed in chloroform, washed with chloroform and eluted with 3% methanol-chloroform. The fraction eluted from 3% methanol-chloroform was evaporated to give 32.1 g (91 percent) of ethyl 1-[$N^G$-nitro-$N^2$-(3-methyl-8-quinoline-sulfonyl)-L-arginyl]-4-methyl-2-piperidinecarboxylate in the form of an amorphous solid.
I.R. (KBr): 3,250, 1,725, 1,640 $cm^{-1}$

(D) 1-[$N^G$-nitro-$N^2$-(3-methyl-8-quinolinesulfonyl)-L-arginyl]-4-methyl-2-piperidinecarboxylic acid

A solution of 30 g of ethyl 1-[$N^G$-nitro-$N^2$-(3-methyl-8-quinolinesulfonyl)-L-arginyl]-4-methyl-2-piperidinecarboxylate in 100 ml of ethanol and 100 ml of 1N sodium hydroxide solution was stirred for 24 hrs. at room temperature. At the end of this period, the solution was neutralized with 1N hydrochloric acid and then concentrated to 70 ml.

The solution was adjusted to pH 11 with 1N sodium hydroxide solution, washed three times with 100 ml of ethyl acetate, acidified with 1N hydrochloric acid and then extracted three times with 100 ml of chloroform. The combined chloroform solution was dried over anhydrous sodium sulfate and evaporated to give 28.0 g (97 percent) of 1-[$N^G$-nitro-$N^2$-(3-methyl-8-quinolinesulfonyl)-L-arginyl]-4-methyl-2-piperidinecarboxylic acid as an amorphous solid.
IR (KBr): 3,300, 1,720, 1,630 $cm^{-1}$
Analysis Calcd. for $C_{23}H_{31}N_7O_7S$ (percent): C, 50.26; H, 5.69; N, 17.84 Found (percent) C, 50.00; H, 5.50; N, 17.49

(E) 1-[$N^2$-(3-methyl-1,2,3,4-tetrahydro-8-quinolinesulfonyl)-L-arginyl]-4-methyl-2-piperidinecarboxylic acid

To a solution of 3.00 g of 1-[$N^G$-nitro-$N^2$-(3-methyl-8-quinolinesulfonyl)-L-arginyl]-4-methyl-2-piperidinecarboxylic acid in 50 ml of ethanol was added 0.5 g of palladium black and then the mixture was shaken under 10 kg/$cm^2$ hydrogen pressure at 100°C for 8 hrs. At the end of this period, the ethanol solution was filtered to remove the catalyst and evaporated to give 2.50 g (90 percent) of 1-[$N^2$-(3-methyl-1,2,3,4-tetrahydro-8-quinolinesulfonyl)-L-arginyl]-4-methyl-2-piperidinecarboxylic acid as an amorphous solid.
IR (KBr): 3,400, 1,620, 1,460, 1,380 $cm^{-1}$
NMR: 100 MHz in $CD_3OD$ $\delta$-value; 6.5 (triplet 1H) 7.1 (doublet 1H), 7.4 (doublet 1H)
Analysis Calcd. for $C_{23}H_{36}N_6O_5S$ (percent): C, 54.31; H, 7.13; N, 16.52 Found (percent) C, 54.01; H, 6.98; N, 16.61

Example 2
*Preparation*

Ethyl (2R,4R)-4-methyl-2-piperidinecarboxylate

*1. Fractionation of trans and cis forms of ethyl 4-methyl-2-piperidinecarboxylate*

Trans and cis forms of ethyl 4-methyl-2-piperidinecarboxylate were fractionated by distillation in vacuo. Trans form; b.p. 83—5°/7 mmHg: Cis form; b.p. 107—8°/5 mmHg.

*2. Optical resolution of trans form*

Racemic ethyl 4-methyl-2-piperidinecarboxylate (trans form) was hydrolyzed by boiling with an excess of conc. HCl for 4 hrs. to give 4-methyl-2-piperidinecarboxylic acid HCl. A desalting of the amino acid HCl was carried out by chromatography using H form ion-exchange resin (Diaion SK—112 manufactured by Mitsubishi Chemical Industries Limited) in usual method to give racemic 4-methyl-2-piperidinecarboxylic acid. To a solution of the racemic amino acid (143.2 g) in boiling 95% ethyl alcohol (2,900 ml) was added L-tartaric acid (150 g). Upon cooling, the precipitated salt (145.9 g) was collected by filtration. The crude crystals were recrystallized from 90% ethyl alcohol (1,000 ml) to give (2R,4R)-4-methyl-2-piperidinecarboxylic acid L-tartaric acid, m.p. 183.9—185.0°C, $[\alpha]_D^{26} = +4.4$ (C = 10% in $H_2O$), Analysis Calcd. for $C_{11}H_{19}NO_8$ (percent): C, 45.05, H, 6.53, N, 4.77 Found (percent): C, 45.12, H, 6.48, N, 4.70. The absolute configuration of the molecule was established by X-ray analysis of the crystal which is a 1:1 complex of the molecule with L-tartaric acid. The product was chromatographed on 2,000 ml of Diaion SK—112 ion-exchange resin packed in water, washed with water and eluted with 3% ammonium hydroxide solution. The fraction eluted from 4% ammonium hydroxide solution was evaporated to dryness to give (2R,4R)-4-methyl-2-piperidinecarboxylic acid (63.0 g) as powdery crystal. Recrystallization of the product from EtOH—$H_2O$ yielded the corresponding amino acid, (2R,4R)-4-methyl-2-piperidinecarboxylic acid, m.p. 275.0—277.8°C, $[\alpha]_D^{18} = -18.0$ (C = 10 2N—HCl), Analysis Calcd. for $C_7H_{13}NO_2$ (percent): C, 58.72, H, 9.15, N, 9.78 Found: C, 58.80, H, 9.09, N, 9.71.

*3. Preparation of ethyl (2R,4R)-4-methyl-2-piperidinecarboxylate*

Thionyl chloride (128.6 g) added dropwise to a stirred suspension of (2R,4R)-4-methyl-2-piperidinecarboxylic acid (51.6 g) in absolute ethyl alcohol (690 ml) below 30°C and stirring continued for 1 hr. at room temperature and then for 1 hr. under reflux. After evaporation of the solvent, the residue was dissolved in benzene (500 ml), washed with 5% $K_2CO_3$ solution (100 ml) and saturated NaCl solution (200 ml), and dried over anhydrous $Na_2SO_4$. Upon evaporation of benzene, the residue was distillated in vacuo to give ethyl (2R,4R)-4-methyl-2-piperidinecarboxylate (57.4 g), b.p. 83—5°C/7

mmHg, $[\alpha]_D^{22} = -24.0$ (C = 5 in EtOH), Analysis Calcd. for $C_9H_{17}NO_2$ (percent): C, 63.13, H, 10.00, N, 8.18 Found (percent): C, 63.20, H, 9.96, N, 8.12.

(A) Ethyl (2R,4R)-1-[$N^G$-nitro-$N^2$-(tert-butoxycarbonyl)-L-arginyl]-4-methyl-2-piperidinecarboxylate

To a stirred solution of 28.3 g of $N^G$-nitro-$N^2$-(tert-butoxycarbonyl)-L-arginine in 450 ml of dry tetrahydrofuran were added in turn 9.0 g of triethylamine and 12.2 g of isobutyl chloroformate while keeping the temperature at −20°C. After 10 minutes, to this was added 15.2 g of ethyl (2R,4R)-4-methyl-2-piperidinecarboxylate and the mixture was stirred for 10 minutes at −20°C. At the end of this period, the reaction mixture was warmed to room temperature. The solvent was evaporated and the residue taken up in 400 ml of ethyl acetate, and washed successively with 200 ml of water, 100 ml of 5% sodium bicarbonate solution, 100 ml of 10% citric acid solution and 200 ml of water. The ethyl acetate solution was dried over anhydrous sodium sulfate.

The solution was evaporated to give 31.3 g (74.5 percent) of ethyl (2R,4R)-1-[$N^G$-nitro-$N^2$-(tert-butoxycarbonyl)-L-arginyl]-4-methyl-2-piperidinecarboxylate in the form of a syrup.
I.R. (KBr): 3,300, 1,730, 1,680 $cm^{-1}$

(B) Ethyl (2R,4R)-1-[$N^G$-nitro-L-arginyl]-4-methyl-2-piperidinecarboxylate hydrochloride

To a stirred solution of 30 g of ethyl (2R,4R)-1-[$N^G$-nitro-$N^2$-(tert-butoxycarbonyl)-L-arginyl]-4-methyl-2-piperidinecarboxylate in 50 ml of ethyl acetate was added 80 ml of 10% dry HCl-ethyl acetate at 0°C. After 3 hours, to this solution was added 200 ml of dry ethyl ether to precipitate a viscous oily product.

This was filtered and washed with dry ethyl ether to give ethyl (2R,4R)-1-[$N^G$-nitro-L-arginyl]-4-methyl-2-piperidinecarboxylate hydrochloride as an amorphous solid.

(C) Ethyl (2R,4R)-1-[$N^G$-nitro-$N^2$-(3-methyl-8-quinoline-sulfonyl)-L-arginyl]-4-methyl-2-piperidinecarboxylate

To a stirred solution of 25 g of ethyl (2R,4R)-1-[$N^G$-nitro-L-arginyl)-4-methyl-2-piperidinecarboxylate hydrochloride in 200 ml of chloroform were added in turn 18.5 g of triethylamine, and 14.7 g of 3-methyl-8-quinolinesulfonyl chloride at 5°C, and stirring was continued for 3 hours at room temperature. At the end of this period, the solution was washed twice with 50 ml of water.

The chloroform solution was dried over anhydrous sodium sulfate. Upon evaporation of the solvent, the residue was chromatographed on 50 g of silica gel packed in chloroform, washed with chloroform and eluted with 3% methanol-chloroform. The fraction eluted from 3% methanol-chloroform was evaporated to give 32.5 g (92.1 percent) of ethyl (2R,4R)-1-[$N^G$-nitro-$N^2$-(3-methyl-8-quinolinesulfonyl)-L-arginyl]-4-methyl-2-piperidinecarboxylate in the form of an amorphous solid.
I.R. (KBr): 3,250, 1,725, 1,640 $cm^{-1}$

(D) (2R,4R)-1-[$N^G$-nitro-$N^2$-(3-methyl-8-quinolinesulfonyl)-L-arginyl]-4-methyl-2-piperidinecarboxylic acid

A solution of 30 g of ethyl (2R,4R)-1-[$N^G$-nitro-$N^2$-(3-methyl-8-quinolinesulfonyl)-L-arginyl]-4-methyl-2-piperidinecarboxylate in 100 ml of ethanol and 100 ml of 1N sodium hydroxide solution was stirred for 24 hrs. at room temperature. At the end of this period, the solution was neutralized with 1N hydrochloric acid and then concentrated to 70 ml.

The solution was adjusted to pH 11 with 1N sodium hydroxide solution, washed with 100 ml of ethyl acetate, and then 100 ml of chloroform, acidified with 1N hydrochloric acid.

The resulting precipitate was filtered and washed with 20 ml of water to give 27 g (95% yield) of (2R,4R)-1-[$N^G$-nitro-$N^2$-(3-methyl-8-quinolinesulfonyl)-L-arginyl]-4-methyl-2-piperidinecarboxylic acid, m.p. 211—213°C.
I.R. (KBr): 3,280, 1,720, 1,620 $cm^{-1}$
Analysis Calcd. for $C_{23}H_{31}N_7O_7S$ (percent): C, 50.26; H, 5.69; N, 17.84 Found (percent) C, 50.05; H, 5.45; N, 17.45

(E) (2R,4R)-1-[$N^2$-(3-methyl-1,2,3,4-tetrahydro-8-quinolinesulfonyl)-L-arginyl]-4-methyl-2-piperidinecarboxylic acid

To a solution of 3.00 g of (2R,4R)-1-[$N^G$-nitro-$N^2$-(3-methyl-8-quinolinesulfonyl)-L-arginyl]-4-methyl-2-piperidinecarboxylic acid in 40 ml of ethanol and 10 ml of acetic acid was added 0.3 g of 5% palladium carbon and then the mixture was shaken under 50 kg/$cm^2$ hydrogen pressure at 80°C for 4 hrs. At the end of this period, the solution was filtered to remove the catalyst and evaporated.

The residual viscous oil shaken with a mixture of 30 ml of chloroform and 30 ml of saturated sodium bicarbonate solution. The chloroform layer was washed with 30 ml of water and evaporated. The resulting crude crystal was recrystallized from ethanol to give 2.6 g (94% yield) of (2R,4R)-1-[$N^2$-(3-methyl-1,2,3,4-tetrahydro-8-quinolinesulfonyl)-L-arginyl]-4-methyl-2-piperidinecarboxylic acid, m.p. 188—191°C.
I.R. (KBr): 3,400, 1,620, 1,460, 1,380 $cm^{-1}$
NMR: 100 MHz in $CD_3OD$ δ-value; 6.5 (triplet 1H) 7.1 (doublet 1H), 7.4 (doublet 1H)

Analysis Calcd. for $C_{23}H_{36}N_6O_5S$ (percent): C, 54.31; H, 7.13; N, 16.52 Found (percent) C, 54.05; H, 6.94; N, 16.65

Example 3

(2R,4R)-1-[$N^2$-(3-methyl-1,2,3,4-tetrahydro-8-quinolinesulfonyl)-L-arginyl]-4-methyl-2-piperidinecarboxylic acid

Starting from ethyl (2R,4R)-1-[$N^G$-nitro-$N^2$-(3-methyl-8-quinolinesulfonyl)-L-arginyl]-4-methyl-2-piperidinecarboxylate which was prepared in Example 2 (C), ethyl (2R,4R)-1-[$N^2$-(3-methyl-1,2,3,4-tetrahydro-8-quinolinesulfonyl)-L-arginyl]-4-methyl-2-piperidinecarboxylate was prepared according to the procedure similar to that described in Example 2 (E).

A mixture of 5 g of ethyl (2R,4R)-1-[$N^2$-(3-methyl-1,2,3,4-tetrahydro-8-quinolinesulfonyl)-L-arginyl]-4-methyl-2-piperidinecarboxylate, 50 ml of ethanol and 50 ml of 1N NaOH aqueous solution was stirred at room temperature for 24 hours. At the end of this period, the reaction mixture was neutralized with 1N HCl aqueous solution and then ethanol was distilled off. The residue was extracted with 50 ml of $CHCl_3$ and washed with water. Upon evaporation of the solvent, the resulting precipitate was filtered and recrystallized from ethanol to give 4.0 g (93% yield) of (2R,4R)-1-[$N^2$-(3-methyl-1,2,3,4-tetrahydro - 8 - quinolinesulfonyl) - L - arginyl] - 4 - methyl - 2 - piperidinecarboxylic acid, m.p. 188—191°C.

Various other $N^2$-arylsulfonyl-L-argininamides were synthesized in accordance with the procedure of the above examples, and the test results are summarized in Table 1.

TABLE 1

| Sample No. | 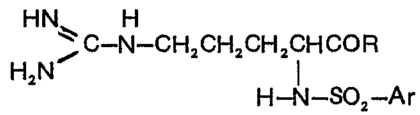 Ar | R | Concentration required to prolong the coagulation time by a factor of two (μm) | Physical properties | Elemental analysis Upper: Calculated Lower: Found C | H | N | I.R. (KBr) ($cm^{-1}$) | N.M.R. δ-value (ppm) ($CD_3OD$) |
|---|---|---|---|---|---|---|---|---|---|
| 1 | 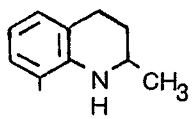 | 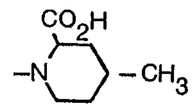 | 0.45 | powder | 54.31<br>54.21 | 7.13<br>6.98 | 16.52<br>16.38 | 3,380, 1,620<br>1,460, 1,375 | 6.5 (t, 1H)<br>7.1 (d, 1H)<br>7.4 (d, 1H) |
| 2 | 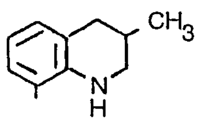 | ,, | 0.08 | ,, | 54.31<br>54.01 | 7.13<br>6.98 | 16.52<br>16.61 | 3,400, 1,620<br>1,460, 1,380 | 6.5 (t, 1H)<br>7.1 (d, 1H)<br>7.4 (d, 1H) |
| 3 | 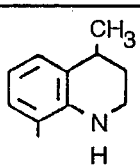 | ,, | 0.2 | ,, | 54.31<br>54.50 | 7.13<br>7.01 | 16.52<br>16.29 | 3,380, 1,620<br>1,380, 1,160 | 6.5 (t, 1H)<br>7.2 (d, 1H)<br>7.4 (d, 1H) |
| 4 | 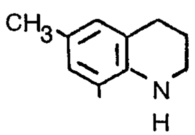 | ,, | 1.8 | ,, | 54.31<br>54.28 | 7.13<br>7.13 | 16.52<br>16.40 | 3,380, 1,620<br>1,380, 1,285 | 6.9 (s, 1H)<br>7.3 (s, 1H) |
| 5 | 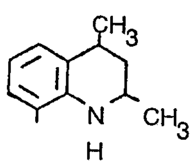 | ,, | 5.5 | ,, | 55.15<br>55.20 | 7.33<br>7.29 | 16.08<br>16.00 | 3,350, 1,620<br>1,380, 1,150 | 6,5 (t, 1H)<br>7.1 (t, 1H)<br>7.4 (t, 1H) |

TABLE 1 (Continued)

| Sample No. | 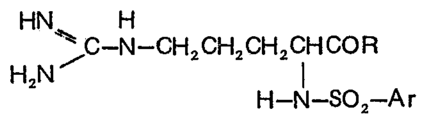 | | Concentration required to prolong the coagulation time by a factor of two (μM) | Physical properties | Elemental analysis Upper: Calculated Lower: Found | | | I.R. (KBr) ($cm^{-1}$) | N.M.R. δ-value (ppm) ($CD_3OD$) |
|---|---|---|---|---|---|---|---|---|---|
| | Ar | R | | | C | H | N | | |
| 6 | 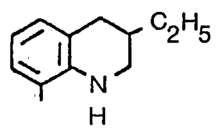 | 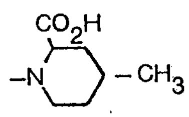 | 0.06 | powder | 55.15<br>55.11 | 7.33<br>7.40 | 16.08<br>15.88 | 3,375, 1,620<br>1,460, 1,380 | 6,6 (t, 1H)<br>7.2 (d, 1H)<br>7.4 (d, 1H) |
| 7 | 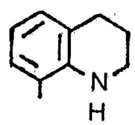 | 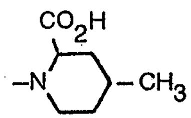 | 0.45 | ,, | 53.42<br>53.12 | 6.93<br>6.63 | 16.99<br>16.59 | 3,380, 1,620<br>1,460, 1,380 | 6.6 (d, 1H)<br>7.1 (d, 1H)<br>7.4 (d, 1H) |
| 8 | 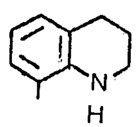 | 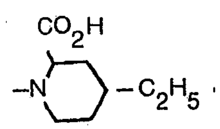 | 0.45 | ,, | 54.31<br>54.20 | 7.13<br>7.19 | 16.52<br>16.41 | 3,380, 1,620<br>1,460, 1,380 | 6.6 (d, 1H)<br>7.1 (d, 1H)<br>7.4 (d, 1H) |
| 9 | 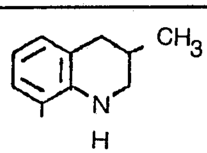 | ,, | | ,, | 55.15<br>55.07 | 7.33<br>7.03 | 16.08<br>16.38 | 3,380, 1,620<br>1,460, 1,380 | 6.5 (t, 1H)<br>7.2 (d, 1H)<br>7.4 (d, 1H) |
| 10 | 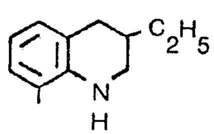 | ,, | | ,, | 55.95<br>55.69 | 7.51<br>7.28 | 15.66<br>15.51 | 3,380, 1,620<br>1,460, 1,380 | 6.5 (t, 1H)<br>7.2 (d, 1H)<br>7.4 (d, 1H) |

TABLE 1 (Continued)

| Sample No. | 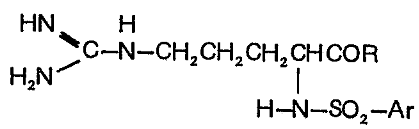 Ar | R | Concentration required to prolong the coagulation time by a factor of two (μM) | Physical properties | Elemental analysis Upper: Calculated Lower: Found C | H | N | I.R. (KBr) ($cm^{-1}$) | N.M.R. δ-value (ppm) ($CD_3OD$) |
|---|---|---|---|---|---|---|---|---|---|
| 11 | 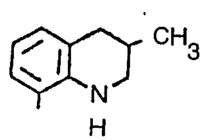 | 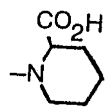 | | powder | 53.42<br>53.28 | 6.93<br>6.63 | 16.99<br>16.69 | 3,380, 1,620<br>1,460, 1,380 | 6.6 (d, 1H)<br>7.1 (d, 1H)<br>7.4 (d, 1H) |
| 12 | 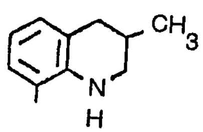 | 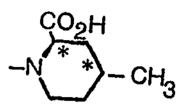 (2R, 4R) | 0,02 | m.p. 188–191 °C | 54.31<br>54.05 | 7.13<br>6.94 | 16.52<br>16.65 | 3,400, 1,620<br>1,460, 1,380 | 6.5 (t, 1H)<br>7.1 (d, 1H)<br>7.4 (d, 1H) |

* represents asymmetric carbon atom

Example 4

Tablets suitable for oral adminstration

Tablets containing the ingredients indicated below may be prepared by conventional techniques.

| Ingredient | Amount per tablet (mg) |
|---|---|
| 1-[$N^2$-(1,2,3,4-tetrahydro-8-quinolinesulfonyl)-L-arginyl]-4-methyl-2-piperidinecarboxylic acid | 250 |
| Lactose | 140 |
| Corn starch | 35 |
| Talcum | 20 |
| Magnesium stearate | 5 |
| Total | 450 mg |

Example 5

Capsules for oral administration

Capsules of the below were made up by thoroughly mixing together batches of the ingredients and filling hard gelatin capsules with the mixture.

| Ingredient | Amount per capsule (mg) |
|---|---|
| 1-[$N^2$-(1,2,3,4-tetrahydro-8-quinolinesulfonyl)-L-arginyl]-4-methyl-2-piperidinecarboxylic acid | 250 |
| Lactose | 250 |
| Total | 500 mg |

Example 6

Sterile solution for infusion

The following ingredients are dissolved in water for intravenous perfusion and the resulting solution is then sterilized.

| Ingredients | Amount (g) |
|---|---|
| 1-[$N^2$-(1,2,3,4-tetrahydro-8-quinolinesulfonyl)-L-arginyl]-4-methyl-2-piperidinecarboxylic acid | 25 |
| Buffer system | As desired |
| Glucose | 25 |
| Distilled water | 500 |

Having now fully described the invention, it will be apparent to one of ordinary skill in the art that many changes and modifications can be made thereto without departing from the spirit of the invention as set forth herein.

**Claims**

1. An $N^2$-arylsulfonyl-L-argininamide of the formula (I):

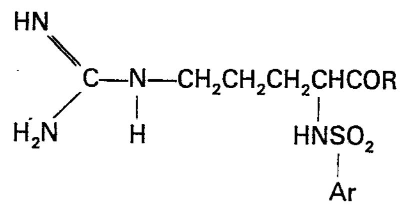

(I)

wherein R is

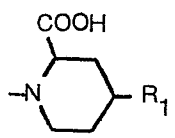

wherein $R_1$ is hydrogen or $C_1$—$C_5$ alkyl; and Ar is 1,2,3,4-tetrahydro-8-quinolyl optionally substituted with at least one $C_1$—$C_5$ alkyl, or the acid addition salt thereof.

2. The compound of Claim 1 wherein $R_1$ is $C_1$—$C_3$ alkyl and Ar is 1,2,3,4-tetrahydro-8-quinolyl optionally substituted with at least one $C_1$—$C_3$ alkyl.

3. The compound of Claim 1 wherein R is (2R,4R)-4-alkyl-2-carboxy-1-piperidino said alkyl being $C_1$—$C_5$ alkyl; and Ar is 1,2,3,4-tetrahydro-8-quinolyl optionally substituted with at least one $C_1$—$C_3$ alkyl.

4. The compound of Claim 3 wherein the alkyl group in the 4-position of the piperidine ring contains 1 to 3 carbon atoms and Ar is 3-alkyl-1,2,3,4-tetrahydro-8-quinolyl said alkyl being $C_1$—$C_3$ alkyl.

5. The compound of Claim 2, which is 1-[$N^2$-(1,2,3,4-tetrahydro-8-quinolinesulfonyl)-L-arginyl]-4-methyl-2-piperidinecarboxylic acid.

6. The compound of Claim 2, which is 1-[$N^2$-(1,2,3,4-tetrahydro-8-quinolinesulfonyl)-L-arginyl]-4-ethyl-2-piperidinecarboxylic acid.

7. The compound of Claim 2, which is 1-[$N^2$-(3-methyl-1,2,3,4-tetrahydro-8-quinolinesulfonyl)-L-arginyl]-4-methyl-2-piperidinecarboxylic acid.

8. The compound of Claim 2, which is 1-[$N^2$-(3-methyl-1,2,3,4-tetrahydro-8-quinolinesulfonyl)-L-arginyl]-4-ethyl-2-piperidinecarboxylic acid.

9. The compound of Claim 2, which is 1-[$N^2$-(3-ethyl-1,2,3,4-tetrahydro-8-quinolinesulfonyl)-L-arginyl]-4-methyl-2-piperidinecarboxylic acid.

10. The compound of Claim 2, which is 1-[$N^2$-(3-ethyl-1,2,3,4-tetrahydro-8-quinolinesulfonyl)-L-arginyl]-4-ethyl-2-piperidinecarboxylic acid.

11. The compound of Claim 4, which is (2R,4R)-1-[$N^2$-(3-methyl-1,2,3,4-tetrahydro-8-quinolinesulfonyl)-L-arginyl]-4-methyl-2-piperidinecarboxylic acid.

12. A process for producing an $N^2$-arylsulfonyl-L-argininamide having the formula (I):

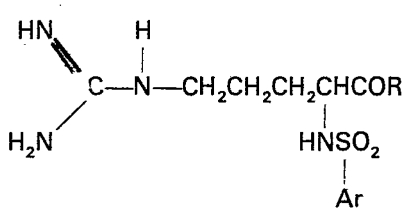

wherein R is

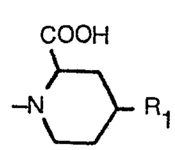

wherein $R_1$ is hydrogen or $C_1$—$C_5$ alkyl; and Ar is 1,2,3,4-tetrahydro-8-quinolyl optionally substituted with at least one $C_1$—$C_5$ alkyl, or the pharmaceutically acceptable acid addition salt thereof, which comprises removing the $N^G$-substituent and, when $R_2$ is aralkyl, said aralkyl group from an $N^G$-substituted-$N^2$-quinolinesulfonyl-L-argininamide having the formula:

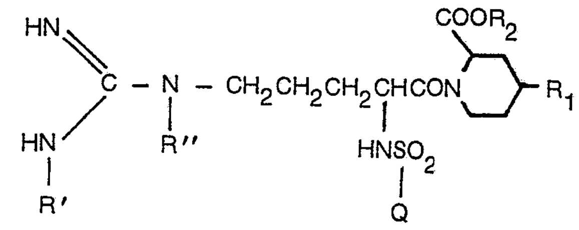

wherein R and Ar are as defined herein above; $R_1$ is as defined in Claim 1; R′ and R″ are selected from the group consisting of hydrogen and protective groups for the guanidino group; and at least one of R′ and R″ is a protective group for the guanidino group; $R_2$ is hydrogen, lower alkyl or aralkyl; and Q is 8-quinolyl optionally substituted with at least one $C_1$—$C_5$ alkyl, which is corresponding to Ar, by means of hydrogenolysis and, at the same time, hydrogenating the quinolyl moiety to the corresponding 1,2,3,4-tetrahydroquinolyl moiety and, when $R_2$ is alkyl, hydrolyzing the ester group at the 2 position of the piperidine ring.

13. A process for producing an $N^2$-arylsulfonyl-L-argininamide having the formula (I):

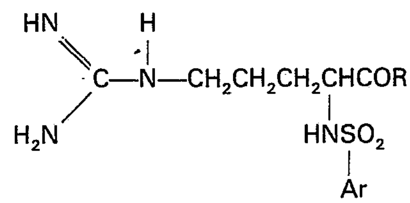

wherein R is

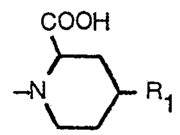

wherein $R_1$ is hydrogen or $C_1$—$C_5$ alkyl; and Ar is 1,2,3,4-tetrahydro-8-quinolyl optionally substituted with at least one $C_1$—$C_5$ alkyl, or the pharmaceutically acceptable acid addition salt thereof, which comprises hydrogenating the quinolyl moiety of the $N^2$-quinolinesulfonyl-L-argininamide having the formula:

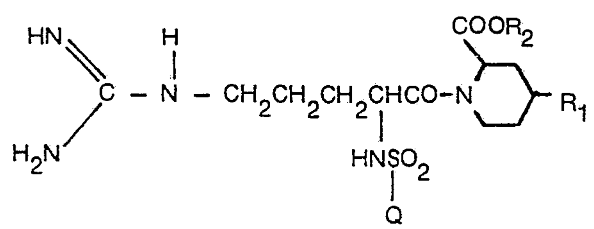

wherein R and Ar are as defined herein above; $R_1$ is as defined in Claim 1; $R_2$ is hydrogen, lower alkyl or aralkyl; and Q is 8-quinolyl optionally substituted with at least one $C_1$—$C_5$ alkyl, which is corresponding to Ar, to the corresponding 1,2,3,4-tetrahydroquinolyl moiety, and at the same time, when $R_2$ is aralkyl, removing said aralkyl group by hydrogenolysis, and when $R_2$ is alkyl hydrolyzing said alkyl ester.

14. A pharmaceutical composition which comprises an antithrombotically effective amount of a compound of Claim 1 and a pharmaceutically acceptable adjuvant.

**Patentansprüche**

1. $N^2$-arylsulfonyl-L-argininamide der allgemeinen Formel I

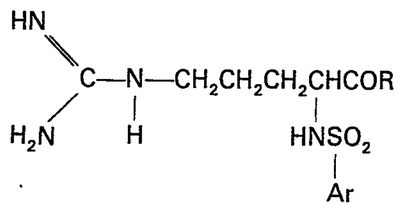

(I)

worin R eine Gruppe der Formel

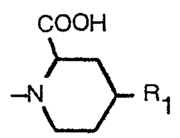

worin $R_1$ für eine Wasserstoffatom oder eine $C_1$—$C_5$-Alkylgruppe steht, und Ar einen gegebenenfalls mit mindestens einer $C_1$—$C_5$-Alkylgruppe substituierten 1,2,3,4-Tetrahydro-8-chinolylrest bedeuten, sowie deren Säureadditionssalze.

2. Verbindungen nach Anspruch 1, dadurch gekennzeichnet, daß $R_1$ eine $C_1$—$C_3$-Alkylgruppe und Ar einen gegebenenfalls mit mindestens einer $C_1$—$C_3$-Alkylgruppe substituierten 1,2,3,4-Tetrahydro-8-chinolylrest bedeuten.

3. Verbindungen nach Anspruch 1, dadurch gekennzeichnet, daß R eine (2R,4R)-4-Alkyl-2-carboxy-1-piperidinogruppe, worin die Alkylgruppe eine $C_1$—$C_5$-Alkylgruppe ist, und Ar einen gegebenenfalls mit mindestens einer $C_1$—$C_3$-Alkylgruppe substituierten 1,2,3,4-Tetrahydro-8-chinolylrest bedeuten.

4. Verbindungen nach Anspruch 3, dadurch gekennzeichnet, daß die Alkylgruppe in der 4-Stellung des Piperidinrings 1 bis 3 Kohlenstoffatome aufweist und Ar einen 3-Alkyl-1,2,3,4-tetrahydro-8-chinolylrest, dessen Alkylgruppe eine $C_1$—$C_3$-Alkylgruppe ist, bedeutet.

5. Verbindung nach Anspruch 2, nämlich 1-[$N^2$-(1,2,3,4-tetrahydro-8-chinolinsulfonyl)-L-arginyl]-4-methyl-2-piperidincarbonsäure.

6. Verbindung nach Anspruch 2, nämlich 1-[$N^2$-(1,2,3,4-tetrahydro-8-chinolinsulfonyl)-L-arginyl]-4-äthyl-2-piperidincarbonsäure.

7. Verbindung nach Anspruch 2, nämlich 1-[$N^2$-(3-methyl-1,2,3,4-tetrahydro-8-chinolinsulfonyl)-L-arginyl]-4-methyl-2-piperidincarbonsäure.

8. Verbindung nach Anspruch 2, nämlich 1-[$N^2$-(3-methyl-1,2,3,4-tetrahydro-8-chinolinsulfonyl)-L-arginyl]-4-äthyl-2-piperidincarbonsäure.

9. Verbindung nach Anspruch 2, nämlich 1-[$N^2$-(3-athyl-1,2,3,4-tetrahydro-8-chinolinsulfonyl)-L-arginyl]-4-methyl-2-piperidincarbonsäure.

10. Verbindung nach Anspruch 2, nämlich 1-[$N^2$-(3-äthyl-1,2,3,4-tetrahydro-8-chinolinsulfonyl)-L-arginyl]-4-äthyl-2-piperidincarbonsäure.

11. Verbindung nach Anspruch 4, nämlich (2R,4R)-1-[$N^2$-(3-methyl-1,2,3,4-tetrahydro-8-chinolinsulfonyl)-L-arginyl]-4-methyl-2-piperidincarbonsäure.

12. Verfahren zur Herstellung eines $N^2$-arylsulfonyl-L-argininamids der allgemeinen Formel I

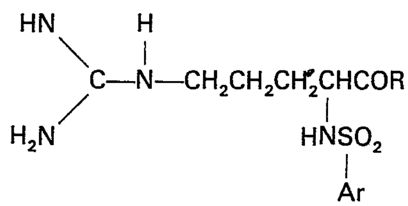

worin R eine Gruppe der Formel

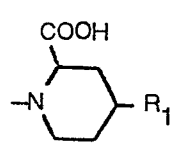

in der $R_1$ für ein Wasserstoffatom oder eine $C_1$—$C_5$-Alkylgruppe steht und Ar einen gegebenenfalls mit

mindestens einer $C_1$—$C_5$-Alkylgruppe substituierten 1,2,3,4-Tetrahydro-8-chinolylrest bedeuten oder der pharmazeutisch annehmbaren Säureadditionssalze davon, dadurch gekennzeichnet, daß man den $N^G$-Substituent und, wenn $R_2$ eine Aralkylgruppe darstellt, diese Aralkylgruppe von einem $N^G$-substituierten $N^2$-Chinolinsulfonyl-L-argininamid der allgemeinen Formel

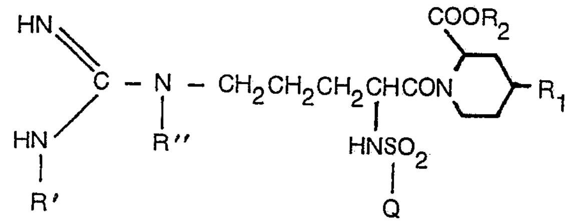

worin R und Ar die oben angegebenen Bedeutungen besitzen, $R_1$ die in Anspruch 1 angegebenen Bedeutungen aufweist, R′ und R″ aus der Wasserstoffatome und Schutzgruppen für die Guanidinogruppe umfassenden Gruppe ausgewählt sind, wobei mindestens eine der Gruppen R′ und R″ eine Schutzgruppe für die Guanidinogruppe darstellt, $R_2$ ein Wasserstoffatom, eine niedrigmolekulare Alkylgruppe oder eine Aralkylgruppe und Q einen der Gruppe Ar entsprechenden, gegebenenfalls mit mindestens einer $C_1$—$C_5$-Alkylgruppe substituierten 8-Chinolylrest bedeuten, durch Hydrogenolyse abspaltet und gleichzeitig den Chinolylrest zu dem entsprechenden 1,2,3,4-Tetrahydrochinolylrest hydriert und, wenn die Gruppe $R_2$ eine Alkylgruppe darstellt, die Estergruppe in der 2-Stellung des Piperidinrings hydrolysiert.

13. Verfahren zur Herstellung des $N^2$-Arylsulfonyl-L-argininamids der allgemeinen Formel I

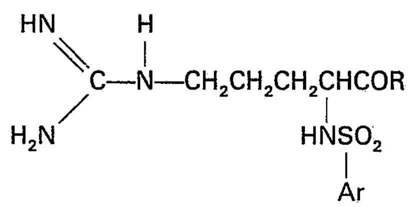

worin R eine Gruppe der Formel

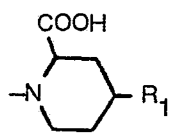

worin $R_1$ für ein Wasserstoffatom oder eine $C_1$—$C_5$-Alkylgruppe steht, und Ar einen gegebenenfalls mit mindestens einer $C_1$—$C_5$-Alkylgruppe substituierten 1,2,3,4-Tetrahydro-8-chinolylrest bedeutet, oder der pharmazeutisch annehmbaren Säureadditionssalze davon, dadurch gekennzeichnet, daß man den Chinolylrest des $N^2$-Chinolinsulfonyl-L-argininamids der allgemeinen Formel

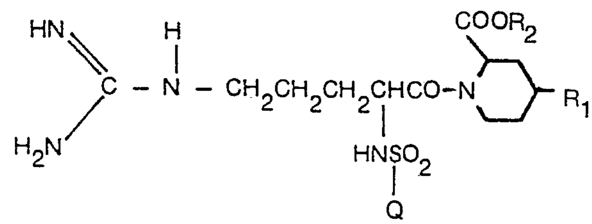

worin R und Ar die oben angegebenen Bedeutungen besitzen, $R_1$ die in Anspruch 1 angegebenen Bedeutungen aufweist, $R_2$ ein Wasserstoffatom, eine niedrigmolekulare Alkylgruppe oder eine Aralkylgruppe und Q einen der Gruppe Ar entsprechenden, gegebenenfalls mit mindestens einer $C_1$—$C_5$-Alkylgruppe substituierten 8-Chinolylrest bedeuten, zu dem entsprechenden 1,2,3,4-Tetrahydrochinolylrest hydriert und gleichzeitig, wenn $R_2$ eine Aralkylgruppe darstellt, diese Aralkylgruppe darstellt, den Alkylester hydrolysiert.

14. Pharmazeutische Zubereitung, enthaltend eine antithrombotisch wirksame Menge einer Verbindung nach Anspruch 1 und einen pharmazeutisch annehmbaren Hilfsstoff.

**Revendications**

1. Un $N^2$-arylsulfonyl-L-argininamide de formule (I):

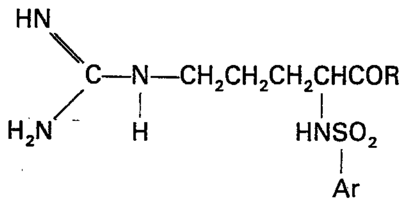

(I)

dans laquelle R représente:

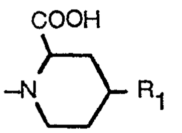

$R_1$ représentant l'hydrogène ou un groupe alkyle en C1—C5; et Ar représente un groupe 1,2,3,4-tétrahydro-8-quinoléyle facultativement substitué par au moins un groupe alkyle en C1—C5, ou le sel d'un tel composé formé par addition avec un acide.

2. Le composé selon la revendication 1, dans lequel $R_1$ représente un groupe alkyle en C1—C3 et Ar le groupe 1,2,3,4-tétrahydro-8-quinoléyle facultativement substitué par au moins un groupe alkyle en C1—C3.

3. Le composé selon la revendication 1, dans lequel R représente le groupe (2R,4R)-4-alkyl-2-carboxy-1-pipéridino dans lequel le radical alkyle est un radical alkyle en C1—C5; et Ar est le groupe 1,2,3,4-tétrahydro-8-quinoléyle facultativement substitué par au moins un groupe alkyle en C1—C3.

4. Le composé selon la revendication 3, dans lequel le groupe alkyle en position 4 du cycle pipéridine contient de 1 à 3 atomes de carbone et Ar représente un groupe 3-alkyl-1,2,3,4-tétrahydro-8-quinoléyle dans lequel le radical alkyle est un radical alkyle en C1—C3.

5. Le composé selon la revendication 2, consistant en l'acide 1-[$N^2$-(1,2,3,4-tétrahydro-8-quinoléine-sulfonyl)-L-arginyl]-4-méthyl-2-pipéridine-carboxylique.

6. Le composé selon la revendication 2, consistant en l'acide 1-[$N^2$-(1,2,3,4-tétrahydro-8-quinoléine-sulfonyl)-L-arginyl]-4-éthyl-2-pipéridine-carboxylique.

7. Le composé selon la revendication 2, consistant en l'acide 1-[$N^2$-(3-méthyl-1,2,3,4-tétrahydro-8-quinoléine-sulfonyl)-L-arginyl]-4-méthyl-2-pipéridine-carboxylique.

8. Le composé selon la revendication 2, consistant en l'acide 1-[$N^2$-(3-méthyl-1,2,3,4-tétrahydro-8-quinoléine-sulfonyl)-L-arginyl]-4-éthyl-2-pipéridine-carboxylique.

9. Le composé selon la revendication 2, consistant en l'acide 1-[$N^2$-(3-éthyl-1,2,3,4-tétrahydro-8-quinoléine-sulfonyl)-L-arginyl]-4-méthyl-2-pipéridine-carboxylique.

10. Le composé selon la revendication 2, consistant en l'acide 1-[$N^2$-(3-éthyl-1,2,3,4-tétrahydro-8-quinoléine-sulfonyl)-L-arginyl]-4-éthyl-2-pipéridine-carboxylique.

11. Le composé selon la revendication 4, consistant en l'acide (2R,4R)-1-[$N^2$-(3-méthyl-1,2,3,4-tétrahydro-8-quinoléine-sulfonyl)-L-arginyl]-4-méthyl-2-pipéridine-carboxylique.

12. Un procédé de préparation d'un $N^2$-arylsulfonyl-L-argininamide ayant la formule (I):

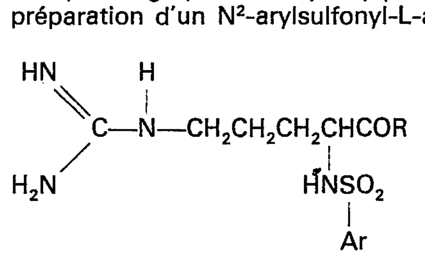

dans laquelle R représente:

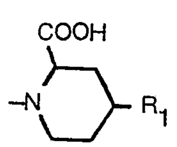

$R_1$ représentant l'hydrogène ou un groupe alkyle en C1—C5; et Ar est un groupe 1,2,3,4-tétrahydro-8-quinoléyle facultativement substitué par au moins un groupe alkyle en C1—C5, ou le sel d'un tel composé

formé par addition avec un acide acceptable pour l'usage pharmaceutique, qui consiste à éliminer le substituant en $N^G$ et, lorsque $R_2$ représente un groupe aralkyle, ce groupe aralkyle, d'un $N^2$-quinoléine-sulfonyl-L-argininamide substitué en $N^G$ et répondant à la formule:

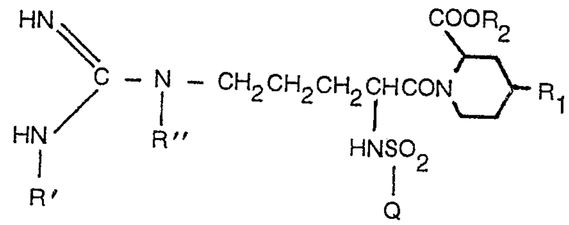

dans laquelle R et Ar ont les significations indiquées ci-dessus, $R_1$ a les significations indiquées dans la revendication 1; R' et R'' son choisis parmi les atomes d'hydrogène et les groupes protecteurs du groupe guanidino; et au moins un des symboles R' et R'' représente un groupe protecteur du groupe guanidino; $R_2$ représente l'hydrogène, un groupe alkyle inférieur ou aralkyle; et Q représente le groupe 8-quinoléyle facultativement substitué par au moins un groupe alkyle en C1—C5, qui correspond à Ar, par hydrogénolyse avec hydrogénation simultanée du radical quinoléyle en le radical 1,2,3,4-tétra-hydro-quinoléyle correspondant, et, lorsque $R_2$ représente un groupe alkyle, hydrolyse du groupe ester en position 2 du cycle pipéridine.

13. Un procédé de préparation d'un $N^2$-arylsulfonyl-L-argininamide répondant à la formule (I):

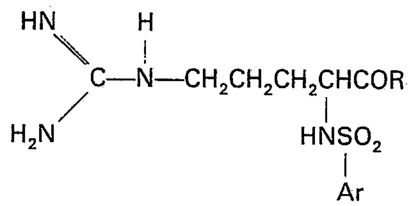

dans laquelle R représente:

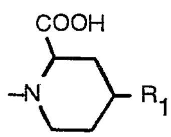

$R_1$ représentant l'hydrogène ou un groupe alkyle en C1—C5; et Ar est un groupe 1,2,3,4-tétrahydro-8-quinoléyle facultativement substitué par au moins un groupe alkyle en C1—C5, ou le sel d'un tel composé formé par addition avec un acide acceptable pour l'usage pharmaceutique, qui consiste à hydrogéner le radical quinoléyle du $N^2$-quinoléine-sulfonyl-L-argininamide répondant à la formule:

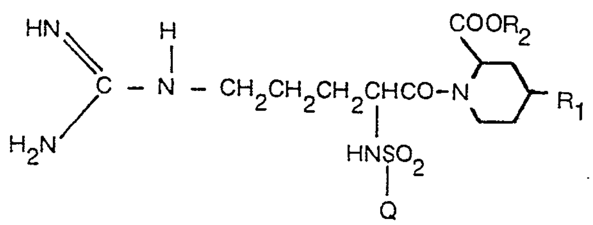

dans laquelle R et Ar ont les significations indiquées ci-dessus, $R_1$ a les significations indiquées dans la revendication 1, $R_2$ représente l'hydrogène, un groupe alkyle inférieur ou aralkyle, et Q représente le groupe 8-quinolèyle facultativement substitué par au moins un groupe alkyle en C1—C5, qui correspond à Ar, en le radical 1,2,3,4-tétrahydroquinoléyle correspondant en éliminant simultanément, lorsque $R_2$ représente un groupe aralkyle, ce groupe aralkyle par hydrogénolyse et, lorsque $R_2$ représente un groupe alkyle, à hydrolyser cet ester alkylique.

14. Une composition pharmaceutique qui comprend une quantité antithrombotique efficace d'un composé de la revendication 1 et un adjuvant acceptable pour l'usage pharmaceutique.